# EUROPÄISCHE PATENTSCHRIFT

(11) **EP 1 618 917 B1**
(45) Veröffentlichungstag und Bekanntmachung des Hinweises auf die Patenterteilung: **10.05.2017**
(21) Anmeldenummer: 05013999.7
(22) Anmeldetag: 29.06.2005
(51) Int. Cl.: A61N 1/05

(54) **Stimulationselektrodenleitung**
Stimulation electrode lead
Sonde de stimulation

(30) Priorität: 23.07.2004 DE 102004036397
(43) Veröffentlichungstag der Anmeldung: 25.01.2006
(73) Patentinhaber: Biotronik CRM Patent AG, 6340 Baar (CH)
(72) Erfinder: Flach, Erhard, 12305 Berlin (DE); Grauhan, Ole, 14163 Berlin (DE); Töllner, Thomas, 12047 Berlin (DE)
(74) Vertreter: Galander, Marcus

(56) Entgegenhaltungen:
- WO-A1-99/11320
- WO-A2-02/18006
- US-A- 5 387 233
- US-A- 5 925 073
- US-A1- 2003 220 677

## Beschreibung

Die Erfindung betrifft eine Stimulationselektrodenleitung zum Anschluss an ein Elektrotherapiegerät, insbesondere ein implantierbares Elektrotherapiegerät wie einen Herzschrittmacher, einen Defibrillator/Kardioverter oder dergleichen. Die Stimulationselektrodenleitung ist ausgebildet, intraluminal in einem Blutgefäß oder im Herzen eines Patienten angeordnet zu werden und weist ein proximales sowie ein distales Ende auf. Am proximalen Ende der Elektrodenleitung ist ein Elektrodenleitungsstecker vorgesehen. Im Bereich ihren distalen Endes trägt die Stimulationselektrodenleitung Stimulationselektroden, welche eine elektrisch leitende Außenoberfläche aufweisen und elektrisch mit dem Elektrodenleitungsstecker verbunden sind, so dass Stimulationsimpulse oder Defibrillationsschocks von dem Elektrotherapiegerät über den Elektrodenleitungsstecker, die Stimulationselektrodenleitung und deren elektrisch leitende Außenoberflächen am distalen Ende abgegeben werden können. Dabei weist die Stimulationselektrodenleitung im Bereich ihres distalen Endes wenigstens einen distalen Elektrodenträgerkörper auf, der wenigstens eine Elektrode trägt und über eine Leitungsbrücke mit einem distalen Ende eines ersten, gestreckten proximalen Elektrodenleitungsabschnitts verbunden ist. Die Leitungsbrücke ist derart vorgeformt ist, dass sie im entspannten Zustand nicht gestreckt und elastisch streckbar ist. Des Weiteren weist der erste, gestreckte Elektrodenleitungsabschnitt der Stimulationselektrodenleitung ein Lumen auf, welches am distalen Ende des ersten, gestreckten Elektrodenleitungsabschnitts in einer distalen Öffnung, durch die ein Führungsdraht aus der Stimulationselektrodenleitung austreten kann, mündet. Daneben weist der distale Elektrodenträgerkörper eine Längsöffnung auf, durch die ein distales Ende eines Führungsdrahtes in den distalen Elektrodenträgerkörper eintreten kann.

Eine solche Stimulationselektrodenleitung ist aus dem Stand der Technik bekannt, wie beispielsweise aus den Patentanmeldungen WO 02/18006 A2 oder US 2003/0220677 A1.

Wenn die Stimulationselektrodenleitung implantiert und an das Elektrotherapiegerät beispielsweise an einen Herzschrittmacher oder Defibrillator angeschlossen ist, werden die vom Herzschrittmacher oder Defibrillator abgegebenen Elektrostimulationsimpulse beispielsweise in der Kammer eines Herzens abgegeben. Diese Kammer ist üblicherweise die rechte Herzkammer, da Stimulationselektrodenleitungen üblicherweise über die obere Hohlvene (Vena Cava Superior) und die rechte Vorhofkammer (rechtes Atrium) des Herzens in die rechte Herzkammer (rechter Ventrikel) eingeführt werden. Mit der Stimulation allein des rechten Ventrikels kann vielen Patienten bereits geholfen werden.

Darüber hinaus sind Zweikammerschrittmacher bekannt, die nicht nur mit Elektroden im rechten Ventrikel zusammenwirken, sondern für die auch im rechten Atrium Elektroden vorgesehen sind, um sowohl das rechte Atrium als auch den rechten Ventrikel stimulieren zu können. Auf diese Weise kann auch solchen Patienten geholfen werden, denen es an einer natürlichen Synchronisation von rechtem Atrium und rechtem Ventrikel mangelt. Beim gesunden Menschen erfolgt, durch den Sinusknoten angeregt, zunächst eine Kontraktion des rechten Atriums. Mit einer gewissen Verzögerungszeit erfolgt anschließend durch atrioventrikuläre Reizleitung eine Kontraktion des rechten Ventrikels. Eine optimale Wirkungsweise des Herzens ergibt sich dann, wenn diese atrioventrikuläre Übergangszeit physiologisch gut eingestellt ist.

Schließlich ist es mittlerweile auch bekannt, nicht nur das rechte Atrium und den rechten Ventrikel eines Herzens zu stimulieren, sondern auch den linken Ventrikel. Dies ist insbesondere für solche Patienten interessant, die an kongestivem Herzversagen (CHF = congestive heart failure) leiden. Im Zusammenhang mit der in solchen Fällen wünschenswerten Stimulation des linken Ventrikels ergibt sich das Problem, dass eine dem linken Ventrikel zugeordnete Elektrodenleitung nicht ohne weiteres zu implantieren ist. Eine an sich bekannte Möglichkeit besteht darin, eine Elektrodenleitung für den linken Ventrikel über die Vena Cava Superior und das rechte Atrium in den Coronar Sinus einzuführen und Elektroden im Coronar Sinus oder einer davon abzweigenden Lateralvene zu platzieren. Da die letztgenannten Blutgefäße im Durchmesser sehr beschränkt sind, sind die konstruktiven Anforderungen an eine entsprechende Elektrodenleitung besonders hoch. Zum einen darf die Elektrodenleitung selbstverständlich den Blutfluss durch diese Blutgefäße nicht in unverträglicher Weise einschränken. Zum anderen besteht immer das Problem, für eine geeignete Fixierung der Elektrodenleitung in diesen Blutgefäßen zu sorgen. Eine sichere Fixierung der Elektrodenleitung ist auch deshalb von Bedeutung, weil eine optimale Einstellung eines Herzschrittmachers immer für einen bestimmten Stimulationsort gefunden werden muss und diese Einstellung nicht beliebig oft wiederholt werden kann.

Bekannte Lösungen für sogenannte Coronar-Sinus-Elektroden (gemeint sind Elektrodenleitungen mit Stimulationselektrodenoberflächen zur Platzierung im Coronar Sinus oder eine davon abzweigende Lateralvene) sind in verschiedener Hinsicht unbefriedigend, so dass nach wie vor der Wunsch nach einer Coronar-Sinus-Elektrode besteht, die gegenüber dem bekannten Stand der Technik jeweils bestimmte Nachteile vermeidet.

Diesem Wunsch wird erfindungsgemäß mit einer Stimulationselektrodenleitung der eingangs genannten Art entsprochen, die im Bereich ihres distalen Endes wenigstens einen distalen Elektrodenträgerkörper aufweist, der wenigstens eine Elektrode trägt und über eine Leitungsbrücke mit einem distalen Ende eines ersten, gestreckten Elektrodenleitungsabschnitts verbunden ist. Des Weiteren ist bei der Stimulationselektrodenleitung am distalen Ende des ersten, gestreckten Elektrodenleitungsabschnitts ein proximaler Elektrodenträgerkörper angeordnet, der die eingangs genannte distale Öffnung aufweist, durch die ein Führungsdraht austreten kann und bei der die Längsöffnung im distalen Elektrodenträgerkörper dergestalt ist, dass ein Führungsdraht durch sie hindurchtreten kann.

Der proximal der Leitungsbrücke befindliche erste Elektrodenleitungsabschnitt schließt ein Lumen ein und kann nach Art herkömmlicher Elektrodenleitungen aufgebaut sein, während die Leitungsbrücke kein Lumen aufzuweisen braucht und gegenüber dem ersten Elektrodenleitungsabschnitt deutlich dünner und vorzugsweise auch beigeweicher als der erste Elektrodenleitungsabschnitt ist, da die Leitungsbrücke im Minimalfall lediglich einen gestreckten, biegeweiche elektrischen Leiter und eine isolierende Hülle beispielsweise aus Kunststoff aufzuweisen braucht.

Der distale Elektrodenträgerkörper am distalen Ende der Leitungsbrücke hat vorzugsweise wieder einen größeren Durchmesser als die Leitungsbrücke, so dass bei der erfindungsgemäßen Elektrodenleitung ein vergleichsweise dicker, ein vergleichsweise dünner und wieder ein vergleichsweise dicker Elektrodenabschnitt in Längsrichtung aufeinander folgen.

Die Leitungsbrücke ist derart geformt, dass sie im entspannten Zustand nicht gestreckt, jedoch elastisch streckbar ist. Die Leitungsbrücke hat mit anderen Worten in ihrem entspannten, vorgeformten Zustand einen irgendwie gebogenen oder gefalteten Zustand und kann aus diesem Zustand heraus elastisch gestreckt werden. Dieses elastische Strecken ist dem in einer Zugfeder ähnlich, mit dem Unterschied, dass die Leitungsbrücke vorzugsweise möglichst vollständig (gerade) zu strecken sein soll, ohne ihre Fähigkeit zu verlieren, anschließend wieder einen gebogenen, gewundenen oder gefalteten Zustand anzunehmen. Letzteres Merkmal ist in Bezug auf die Fixation der Elektrodenleitung im jeweiligen Blutgefäss gewünscht. Die beanspruchte Elektrodenleitung lässt sich für das Einführen in das Blutgefäss möglichst vollständig strecken und erleichtert auf diese Weise das Einführen. Einmal an ihren Platz gebracht, wird zugelassen, dass sich die Elektrodenleitung entspannt und eine gewundene, gebogene oder gefaltete Form annimmt, mit der sich die Elektrodenleitung im Blutgefäß verankert.

Die der Elektrodenleitung zuvor aufgeprägte Form, die diese im entspannten Zustand anzunehmen sucht, kann in einer bevorzugten Ausführungsvariante durch eine elastische, vorgeformte Kunststoffhülle vorgegeben werden, die einen elektrischen Leiter umhüllt. Der elektrische Leiter kontaktiert die Elektrode auf dem distalen Elektrodenträgerkörper und verbindet diesen mit dem Elektrodenleitungsstecker am proximalen Ende der Elektrodenleitung.

Bevorzugte Gestalten der vorgeformten Leitungsbrücke sind eine Schlangenform oder eine Helixform.

Die Leitungsbrücke hat im Übrigen vorzugsweise einen deutlich geringeren Durchmesser als der distale Elektrodenträgerkörper.

Die Stimulationselektrodenleitung kann in einer bevorzugten Ausführungsvariante am distalen Ende des ersten, gestreckten Elektrodenleitungsabschnitts einen zweiten, proximalen Elektrodenträgerkörper aufweisen. Dieser hat vorzugsweise ebenfalls einen größeren Durchmesser als die Leitungsbrücke und als der wenigstens unmittelbar an den proximalen Elektrodenträgerkörper anschließende Abschnitt des ersten, gestreckten Elektrodenleitungsabschnitts. Der proximale Elektrodenträgerkörper ist somit zwischen dem ersten, gestreckten Elektrodenleitungsabschnitt und der Leitungsbrücke angeordnet. Die Leitungsbrücke verbindet den proximalen Elektrodenträgerkörper mit dem distalen Elektrodenträgerkörper. Die Stimulationselektrodenleitung endet am distalen Elektrodenträgerkörper. Die Elektrodenträgerkörper haben vorzugsweise den annähernd gleichen Außendurchmesser.

Im proximalen Elektrodenträgerkörper ist vorzugsweise eine distale Öffnung vorgesehen, durch die ein Führungsdraht oder ein Implantationswerkzeug ähnlich einem Führungsdraht aus der Stimulationselektrodenleitung austreten kann. Im distalen Elektrodenträgerkörper ist vorzugsweise eine Längsöffnung vorgesehen, durch die ein solcher Führungsdraht ein- und vorzugsweise hindurchtreten kann. Diese erlaubt es, den distalen Elektrodenleitungskörper, der mit der übrigen Stimulationselektrodenleitung praktisch nur durch eine relativ dünne, flexible Leitungsbrücke verbunden ist, auf einen relativ steiferen Führungsdraht aufzufädeln.

Die Längsöffnung im distalen Elektrodenträgerkörper kann einen gestuften Innendurchmesser haben, der in distaler Richtung kleiner ist, als in der dem proximalen Elektrodenträgerkörper zugewandten Richtung. So kann beispielsweise ein Führungsdraht oder Implantationswerkzeug vollständig durch den distalen Elektrodenträgerkörper hindurchgeführt werden. Auf den Führungsdraht kann dann eine Hülse oder ein Schubschlauch aufgeschoben werden, deren bzw. dessen Außendurchmesser nicht mehr durch die Längsöffnung in dem distalen Elektrodenträgerkörper passt und die dazu genutzt werden kann, die Leitungsbrücke mittels eines durch die Hülse oder den Schubschlauch vermittelten Schubes zu strecken. Um dies zu bewirken, reicht es aus, wenn die Längsöffnung im distalen Elektrodenträgerkörper einen geringeren Innendurchmesser aufweist als die distale Öffnung im proximalen Elektrodenträgerkörper. Der engste Durchmesser der Längsöffnung im distalen Elektrodenträgerkörper ist so bemessen, dass er das Hindurchführen eines Führungsdrahtes gerade erlaubt, nicht jedoch das Hindurchführen einer auf den Führungsdraht aufgeschobenen Hülse.

Ein geeignetes Implantationswerkzeug umfasst vorzugsweise mindestens einen Schlauch, der ein Lumen aufweist, das es erlaubt, den Schlauch über einen herkömmlichen Führungsdraht zu schieben. Der Schlauch, im folgenden auch Schubschlauch genannt, soll eine ausreichende Steifigkeit besitzen, um die Elektrodenträgerkörper vorschieben zu können. Zum Ankoppeln des Schubschlauches an beispielsweise den distalen Elektrodenträgerkörper kann die Längsöffnung in dem distalen Elektrodenträgerkörper wenigstens teilweise einen Durchmesser haben, der geringer ist als der Außendurchmesser des Schubschlauches, sodass der Schubschlauch wenigstens nicht vollständig durch die Längsöffnung hindurch zu führen ist, sondern den distalen Elektrodenträgerkörper vielmehr mitnimmt, wenn der Schubschlauch vorgeschoben wird. Die Längsöffnung in dem distalen Elektrodenträgerkörper sollte auf jeden Fall so bemessen sein, dass der eigentliche Führungsdraht vollständig durch den Elektrodenträgerkörper hindurch zu führen ist.

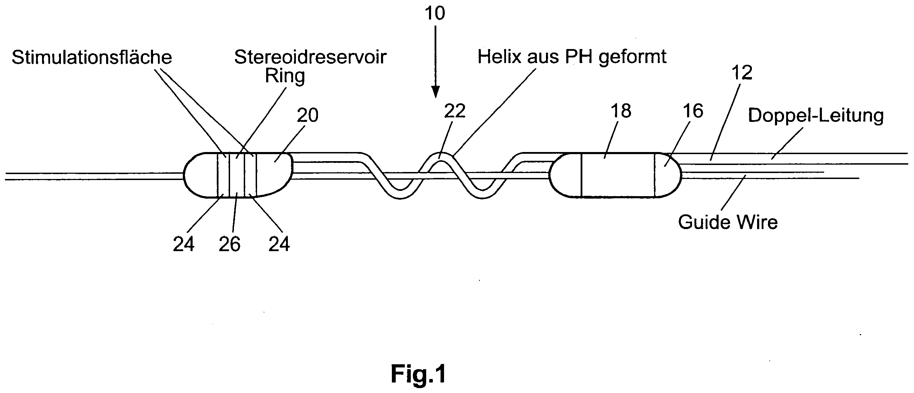

Das Implantationswerkzeug kann in dieser Ausführungsvariante einen zweiten Schubschlauch größeren Durchmessers aufweisen, der über den zuvor genannten Schubschlauch zu schieben ist und der wenigstens nicht vollständig durch die Längsöffnung im proximalen Elektrodenträgerkörper hindurchtreten kann, um auf diese Weise den proximalen Elektrodenträgerkörper mitnehmen zu können. Eine weitergehende Beschreibung dieser Ausführungsvariante ist der Figurenbeschreibung zu den Figuren 1 bis 6 zu entnehmen.

In einer alternativen Ausführungsvariante ist ein doppelwandiger Schubschlauch vorgesehen, der an seinem distalen Ende einen expandierbaren Ballon trägt. Dieser Ballon kann mittels eines Fluids expandiert werden, welches durch die Doppelwand des Schubschlauches in den Ballon zu leiten ist. Die Doppelwand des Schubschlauches wird von einem inneren und einem äußeren Schubschlauch gebildet, die zwischen sich einen entsprechenden Freiraum einschließen, durch den das Fluid zum Expandieren des Ballons zu leiten ist. Ein Koppeln eines derartigen doppelwandigen Schubschlauchs mit Ballon am distalen Ende mit einer der beiden Elektrodenträgerkörper kann dadurch erfolgen, dass der Ballon am distalen Ende des doppelwandigen Schubschlauches in eine der Längsöffnungen der beiden Elektrodenträgerkörper eingeführt wird und dann expandiert wird, sodass der doppelwandige Schubschlauch und der entsprechende Elektrodenträger auf Grund des Expansion des Ballons durch Kraft- oder Formschluss miteinander gekoppelt sind. Diese zu der zuvor beschriebenen Ausführungsvariante eines Implantationswerkzeuges mit zwei unabhängigen Schubschläuchen alternative Ausführungsvariante eines Implantationswerkzeuges ist auf Grund des erforderlichen Ballons zwar aufwendiger, erlaubt es aber, die Elektrodenträgerkörper mittels des doppelwandige Schubschlauches nicht nur vorzuschieben, sondern - falls gewünscht - auch zurückzuziehen. Zur Implantation der Elektrodenleitung wird der Ballon des doppelwandigen Schubschlauches mit dem distalen Elektrodenträgerkörper gekoppelt, um dann den distalen Elektrodenträgerkörper entlang eines zuvor eingeführten Führungsdrahtes vorschieben zu können. Dabei wird die Leitungsbrücke zwischen den beiden Elektrodenträgerkörpern gestreckt, sodass die Leitungsbrücke sich nicht mit Gefäßwänden verklemmen kann.

Die beiden Elektrodenträgerkörper sind vorzugsweise als Kunststoff- oder Keramikkörper ausgebildet und sind damit sowohl körperverträglich als auch elektrisch isolierend. Der jeweilige Elektrodenträgerkörper hat vorzugsweise eine Länge zwischen 1 - 3 mm.

Mit den zuvor erwähnten Maßnahmen wird bezweckt, dass ein distaler Endbereich des ersten, gestreckten Elektrodenleitungsabschnitts, der oder die Elektrodenträgerkörper und die Leitungsbrücke im Coronar Sinus oder einer vom Coronar Sinus abzweigenden Lateralvene platziert werden können. Dieser Zweck definiert in einer bevorzugten Ausführungsvariante die Dimensionen der Bestandteile der beanspruchten Stimulationselektrodenleitungen.

Die Erfindung schließt auch ein Gesamtsystem ein, welches neben der zuvor beschriebenen Elektrodenleitung auch ein Implantationswerkzeug der zuvor beschriebenen Art umfasst.

Die Erfindung soll nun anhand eines Ausführungsbeispieles mit Hilfe der Figuren näher erläutern werden. Von den Figuren zeigen
- Figur 1: einen Überblick über das distale Ende einer erfindungsgemäßen Stimulationselektrodenleitung;
- Figur 2: eine schematische Darstellung eines Führungsdrahtes mit jeweils einem aufgeschobenen inneren und äußeren Schubschlauch als Schubhülsen für die Implantation der in Figur 1 dargestellten Stimulationselektrodenleitungen;
- Figur 3: eine Skizze der Elektrodenleitung in zwei unterschiedlichen Zuständen, nämlich einmal mit gestreckter Leitungsbrücke und einmal mit entspannter Leitungsbrücke;
- Figur 4: den distalen Elektrodenleitungskörper in einem Längsschnitt;
- Figuren 5a und b: den proximalen Elektrodenträgerkörper in Schnittdarstellungen;
- Figur 6: ein Detail eines distalen Endes eines geeigneten Schubschlauches;
- Figur 7: eine alternative Ausführungsvariante eines distalen Endes einer Elektrodenleitung mit alternativem Implantationswerkzeug; und
- Figur 8: eine schematische Darstellung eines proximalen und des distalen Endes des Implantationswerkzeugs aus Figur 7.

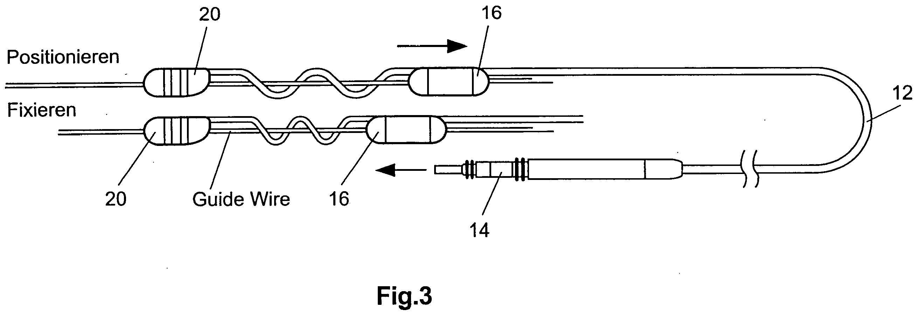

Figur 1 gibt einen Überblick über das distale Ende einer erfindungsgemäßen Stimulationselektrodenleitung 10. Zu erkennen ist ein proximaler Leitungsabschnitt 12, der sich über den rechten Rand der Darstellung und Figur 1 hinaus bis zu einem Elektrodenleitungsstecker 14 an einem proximalen Ende der Elektrodenleitung erstreckt; siehe auch Figur 3. In dem proximalen Elektrodenleitungsabschnitt 12 sind wenigstens zwei elektrische Leiter angeordnet, über die Elektroden am distalen Ende der Elektrodenleitung 10 elektrisch mit entsprechenden Kontaktflächen des Elektrodenleitungssteckers 14 verbunden sind. Der ersteproximale Elektrodenleitungsabschnitt 12 kann in bekannter Weise aufgebaut sein, dass heißt, beispielsweise eine Helixwendel aus Draht umfassen, die von einer isolierenden und dichtenden Kunststoffhülle umgeben ist. Vorzugsweise umfasst er zwei elektrisch leitende Seile, die von einer isolierenden Hülle aus Polyurethan umgeben sind.

Am distalen Ende des proximalen Elektrodenleitungsabschnitts 12 ist ein erster Elektrodenträgerkörper 16 angeordnet, der eine großflächige Stimulationselektrode 18 mit elektrisch leitender Außenoberfläche aufweist.

Distal des ersten Elektrodenträgerkörpers 16 ist ein zweiter, distaler Elektrodenträgerkörper 20 angeordnet, der über eine helixartig vorgeformte Leitungsbrücke 22 mit geringem Außendurchmesser mit dem ersten Elektrodenträgerkörper 16 verbunden ist.

Der zweite, distale Elektrodenträgerkörper 20 besitzt eine zweite Stimulationselektrode mit zwei elektrisch leitenden Stimulationsflächen 24, zwischen denen ein Steroidreservoir 26 in Form eines Rings angeordnet ist. Die zweite Stimulationselektrode mit den Stimulationsflächen 24 ist durch einen elektrischen Leiter innerhalb der Leitungsbrücke 22 und dem proximalen Elektrodenleitungsabschnitt mit einer Kontaktfläche des Elektrodensteckers 14 verbunden.

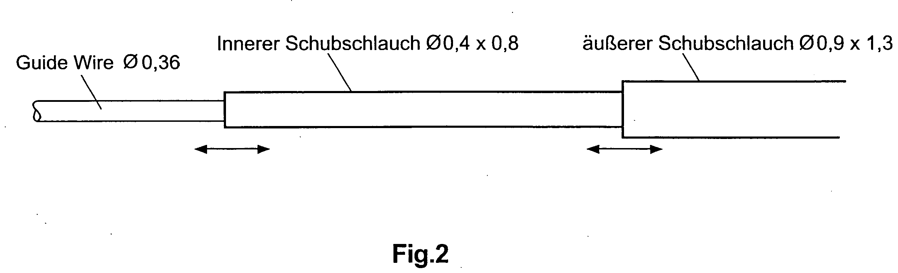

Beide Elektrodenträgerkörper, der erste proximale Elektrodenträgerkörper 16 und der zweite, distale Elektrodenträgerkörper 20 besitzen jeweils eine zentrale Längsbohrung, wobei der Durchmesser der zentralen Längsbohrung im zweiten, distalen Elektrodenträgerkörper 20 wenigstens über eine Teillänge der Längsbohrung kleiner ist, als die Längsbohrung im ersten, proximalen Elektrodenträgerkörper. Die Längsbohrungen in den beiden Elektrodenträgerkörpern 16 und 20 dienen der Aufnahme eines Führungsdrahtes 30, der in Figur 1 ebenfalls dargestellt ist. Außerdem ist der Führungsdraht 30 in Figur 2 separat abgebildet.

Figur 2 ist zu entnehmen, dass der Führungsdraht 30 dreiteilig aufgebaut ist, nämlich einen Führungsdraht im engeren Sinne 32 mit einem Durchmesser von 0,36 mm, auf welchen ein innerer Schubschlauch aufgeschoben ist, der einen Innendurchmesser (inneres Lumen) von 0,4 mm besitzt und einen Außendurchmesser von 0,8 mm. Auf diesen inneren Schubschlauch 34 ist ein äußerer Schubschlauch 36 aufgeschoben, der ein Lumen mit einem Innendurchmesser von 0,9 mm besitzt und der einen Außendurchmesser von 1,3 mm besitzt. Der Führungsdraht im engeren Sinne 32 ist aufgebaut und steuerbar wie bekannte Führungsdrähte. Der innere Schubschlauch 34 ist so bemessen, dass er durch die Längsbohrung im ersten, proximalen Elektrodenträgerkörper 16 vollständig hindurchgeführt werden kann, jedoch nicht durch die Längsbohrung im zweiten, distalen Elektrodenträgerkörper 20. Lediglich der Führungsdraht im engeren Sinne 32 kann vollständig durch die Längsbohrung des zweiten, distalen Elektrodenträgerkörpers 20 hindurchgeführt werden. Die Längsbohrung im ersten, proximalen Elektrodenträgerkörper 16 ist so bemessen, dass zwar der innere Schubschlauch 34 vollständig durch diese Längsbohrung hindurch geführt werden kann, nicht jedoch der äußere Schubschlauch 36.

Dies erlaubt es, zunächst den Führungsdraht im engeren Sinne 32 durch beide Elektrodenträgerkörper 16 und 20 hindurchzufädeln, so dass der Führungsdraht im engeren Sinne 32 eine Führung für beide Elektrodenträgerkörper bildet. Um beide Elektrodenträgerkörper 16 und 20 entlang des Führungsdrahtes im engeren Sinne 32 vorzuschieben, werden innerer Schubschlauch 34 und äußerer Schubschlauch 36 synchron vorgeschoben. Der zweite, distale Elektrodenträgerkörper 20 wird dabei durch den inneren Schubschlauch 34 bewegt, da dieser innere Schubschlauch 34 nicht vollständig durch die Längsbohrung im zweiten, distalen Elektrodenträgerkörper 20 hindurchgeführt werden kann. Der erste, proximale Elektrodenträgerkörper 16 wird durch den äußeren Schubschlauch 36 vorgeschoben.

Beim Vorschieben der beiden Schubschläuche 34 und 36 zum Einführen der Elektrodenleitung 10 werden die beiden Schubschläuche so zueinander positioniert, dass die Leitungsbrücke 22 im wesentlichen gestreckt ist. Dies ist in Figur 3a dargestellt. Nachdem auf diese Weise insbesondere der zweite, distale Elektrodenträgerkörper 20 am richtigen Ort positioniert ist, wird nur noch der äußere Schubschlauch 36 vorgeschoben, und nicht mehr der innere Schubschlauch 34. Dies führt dazu, dass der erste, proximale Elektrodenträgerkörper 16 näher an den zweiten distalen Elektrodenträgerkörper 20 herangeschoben wird, so dass die Leitungsbrücke 22 nicht mehr wie in Figur 3 im wesentlichen gestreckt ist, sondern ihre ursprünglich vorgeformte, helixartig gewundene Form annimmt. Diese helixartig gewundene Form der Leitungsbrücke 22 (siehe Figur 3b) führt dazu, dass die beiden Elektrodenträgerkörper 16 und 20 durch die sich an eine Gefäßwand anlegende Leitungsbrücke in ihrer Position fixiert sind.

Es sei darauf hingewiesen, dass bei der in den Figuren 1 - 3 dargestellten Ausführungsform beide Elektrodenträgerkörper 16 und 20 einen Durchmesser aufweisen, der mehr als doppelt so groß ist wie der Durchmesser des proximalen Elektrodenleitungsabschnitts 12. Die Bohrung im ersten, proximalen Elektrodenträgerkörper 16 besitzt zwei Mündungen am proximalen und am distalen Ende des ersten, proximalen Elektrodenträgerkörpers 16. Dies erlaubt es, den Führungsdraht 30 neben dem Ort, an dem der proximale Elektrodenleitungsabschnitt 12 am ersten Elektrodenträgerkörper 16 ansetzt, in die Längsbohrung in dem ersten Elektrodenträgerkörper 16 einzuführen.

Alternativ ist es auch möglich, im proximalen Elektrodenleitungsabschnitt 12 ein Führungsdrahtlumen vorzusehen, welches direkt in dem ersten Elektrodenträgerkörper mündet, so dass der Führungsdraht 30 im inneren des proximalen Elektrodenleitungsabschnitts 12 geführt werden kann. Obwohl diese Ausführungsvariante möglicherweise auch Vorteile mit sich bringt, wird die in Figuren 1 - 3 dargestellte Ausführungsvariante bevorzugt, da der Führungsdraht 30 mit seinen zwei aufgeschobenen Schubschläuchen 34 und 36 einen größeren Durchmesser hat als ein üblicher Führungsdraht im engeren Sinne. Außerdem werden durch die in Figuren 1 - 3 dargestellte Ausführungsvariante solche Probleme vermieden, die daraus resultieren könnten, dass Blut eines Patienten in ein Führungsdrahtlumen im proximalen Elektrodenleitungsabschnitt eindringt.

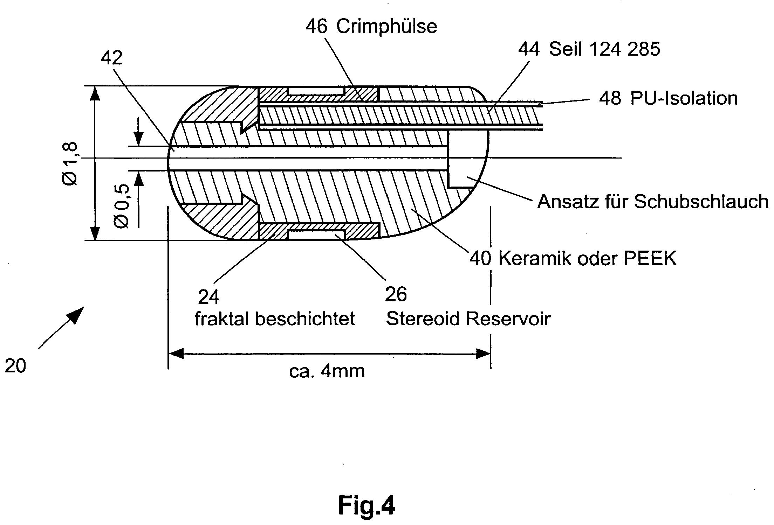

Figur 4 zeigt einen Längsschnitt durch den distalen Elektrodenträgerkörper, die der Ausführungsvariante in den Figuren 1 - 3 entspricht. Wie Figur 4 zu entnehmen ist, besitzt der distale Elektrodenträgerkörper 20 einen Grundkörper aus Keramik oder PEEK (Polyetheretherketone). Der Grundkörper 40 ist mit einer Längsbohrung 42 versehen, die abgestuft ist und im distalen Bereich des Grundkörpers 40 einen Innendurchmesser von 0,5 mm besitzt und am proximalen Ende einen größeren Durchmesser als Ansatz für den inneren Schubschlauch 34. Der Grundkörper 40 trägt eine Ringelektrode als distale Elektrode mit fraktal beschichteten Elektrodenoberflächen 24. In der distalen Elektrode ist eine Ringnut vorgesehen, die das Steroidreservoir 26 aufnimmt. Die distale Elektrode ist über ein elektrisch leitendes Seil 44 und eine auf das distale Ende des Seils 44 aufgekrimpte Krimphülse 46 elektrisch kontaktiert. Das Seil 44 verläuft proximal innerhalb einer isolierenden Hülle 48 aus Polyurethan und bildet mit dieser zusammen die Leitungsbrücke 22.

Der distale Elektrodenträgerkörper 20 besitzt eine Länge von ca. 4 mm und einen Außendurchmesser von etwa 1,8 mm.

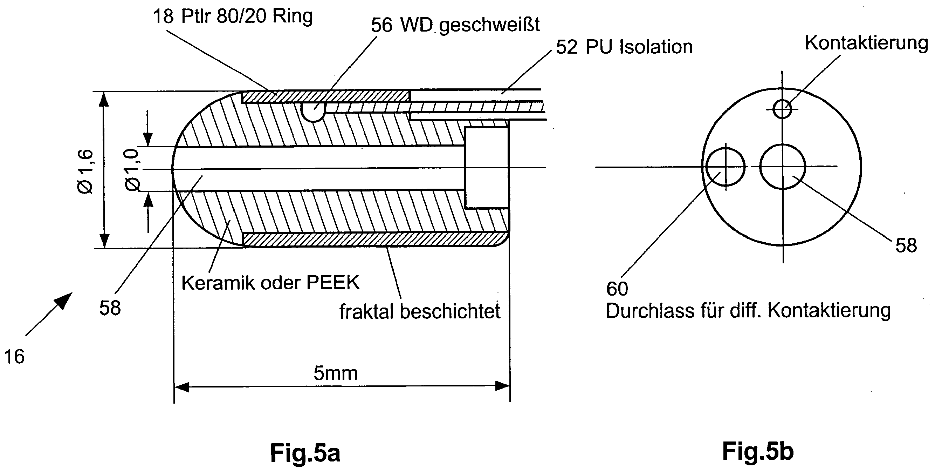

Figur 5 zeigt den ersten, proximalen Elektrodenträgerkörper 16 im Längs- und Querschnitt. Auch der Elektrodenträgerkörper 16 besitzt einen Grundkörper 50 aus Keramik oder PEEK. Der Grundkörper 50 trägt eine großflächige Ringelektrode 18, die aufgrund ihrer Fläche nach Implantation als indifferente Elektrode wirkt.

An dem proximalen Elektrodenträgerkörper 16 ist der proximale Elektrodenleitungsabschnitt 12 direkt angeschlossen. Auch dieser proximale Elektrodenleitungsabschnitt 12 besitzt eine isolierende Hülle 52 aus Polyurethan, die zwei elektrisch leitende Seile einschließt, von denen ein zweites elektrisch leitendes Seil 54 in Figur 5 dargestellt ist.

Das zweite elektrisch leitende Seil 54 ist über eine Schweißverbindung 56 an die Elektrode 18 angeschlossen.

Die Elektrode 18 ist aus einer Platin-Iridiumlegierung 80/20 hergestellt.

In dem Grundkörper 50 ist eine Längsbohrung 58 vorgesehen, die in ihrem distalen Bereich einen Innendurchmesser von 1,9 mm aufweist und in ihrem proximalen Bereich einen Ansatz größeren Durchmessers hat, der der Aufnahme des distalen Endes des äußeren Schubschlauches 36 dient. Figur 5b zeigt einen Querschnitt durch den proximalen Elektrodenträgerkörper 16, in dem eine Bohrung 60 zu erkennen ist, die dem Anschluss der Leitungsbrücke 22 und damit der Kontaktierung der distalen Elektrodenflächen 24 dient. Der proximale Elektrodenträgerkörper 16 besitzt eine Länge von etwa 5 mm und hat einen Außendurchmesser von etwa 1,6 mm.

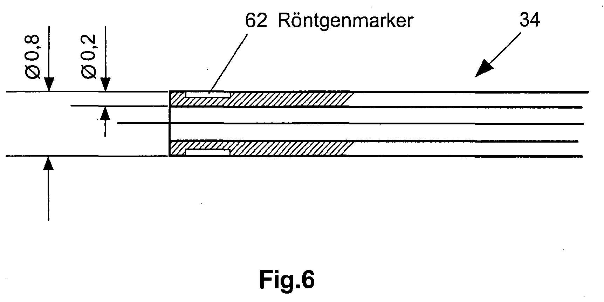

Figur 6 zeigt das distale Ende des inneren Schubschlauches 34. Zu erkennen ist, dass der innere Schubschlauch 34 an seinem distalen Ende einen Röntgenmarker 62 in Form eines Ringes aus röntgensichtbarem Material, beispielsweise Gold, aufweist. Der Röntgenmarker 62 dient der genauen Positionierung des inneren Schubschlauches 34 und damit der distalen Elektrode 20.

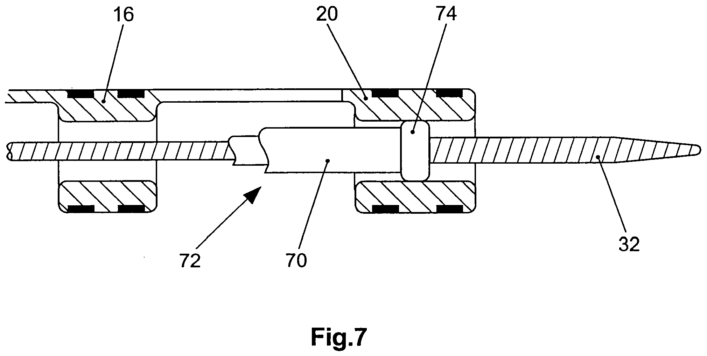

Figur 7 zeigt das distale Ende einer Stimulationselektrodenleitung ähnlich der aus den Figuren 1 bis 6 mit einem alternativen Implantationswerkzeug 70, welches einen doppelwandigen Schubschlauch 72 mit einem expandierbaren Ballon 74 an seinem distalen Ende aufweist. Der doppelwandige Schubschlauch 72 wird von einem inneren Schubschlauch 72a und einem äußeren Schubschlauch 72b gebildet. Zwischen diesen besteht ein Freiraum, durch den ein Fluid in den Ballon 74 zu leiten ist, um diesen zu expandieren. Der innere Schubschlauch 72a weist im übrigen ein Lumen auf, um den doppelwandigen Schubschlauch 72 über einen Führungsdraht 32 schieben zu können.

Die Elektrodenträgerkörper 16 und 20 aus Figur 7 unterscheiden sich von den Elektrodenträgerkörpern 16 und 20 aus den Figuren 1 bis 5 dadurch, dass die jeweiligen Längsbohrungen bei beiden Elektrodenträgerkörpern 16 und 20 den gleichen, durchgehenden Durchmesser aufweisen.

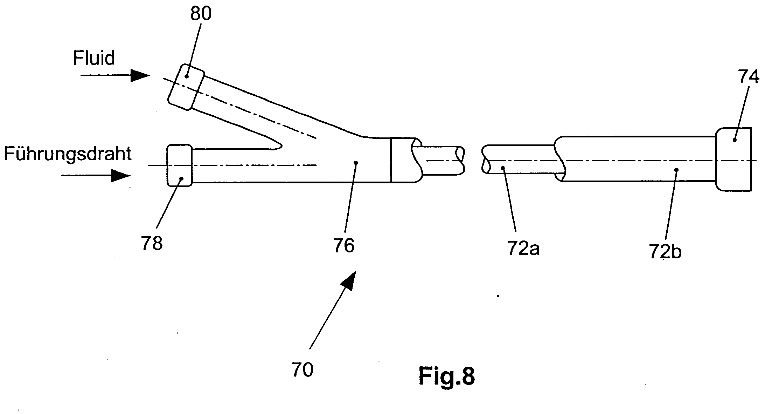

In Figur 8 ist dargestellt, wie ein proximales Ende des Implantationswerkzeuges 70 aufgebaut sein kann. Der doppelwandige Schubschlauch 70 mündet in einer Y-Luerkupplung 76 die einen ersten Port 78 aufweist, der mit dem vom inneren Schubschlauch 72a eingeschlossenen Lumen für den Führungsdraht in Verbindung steht und durch den somit ein Führungsdraht eingeschoben werden kann. Ein zweiter Port 80 steht mit dem zwischen innerem Schubschlauch 72a und äußeren Schubschlauch 72b eingeschlossenen Zwischenraum in Verbindung und dient dazu, Fluid in den Ballon 74 zu leiten, um den Ballon 74 zu expandieren.

Der Ballon 74 besteht aus einem elastischen Material, sodass er mittels eines in den Ballon 74 unter Druck eingeleiteten Fluids zu expandieren ist und wieder zu seinem ursprünglichen, geringeren Außendurchmesser zurückkehrt, sobald der Fluiddruck nachlässt. Ein als Fluid geeignetes Medium ist beispielsweise eine Infusionslösung, die körperverträglich ist, sodass Leckagen unproblematisch sind. An dem zweiten Port 80 der Luerkupplung 76 kann eine Pumpe angeschlossen werden, mit der der Flüssigkeitsdruck im Ballon 74 geändert werden kann.

Das Implantationswerkzeug 70 kann visuelle Längenmarkierungen im proximalen Bereich oder zusätzlich oder alternativ Röntgenmarker im distalen Bereich besitzen, um einen Indikator für die relative axiale Position von Stimulationselektrodenleitung und Implantationswerkzeug zu bieten. Damit ist es möglich, den Ballon 74 des Implantationswerkzeuges innerhalb der gewünschten elektrisch-aktiven Elektrodenoberflächen der Stimulationselektrodenleitung zu platzieren.

Der von dem doppelwandigen Schlauch 72 gebildete Schaft des Implantationswerkzeuges 70 ist aus einem ausreichend stabilen Schlauchmaterial hergestellt, um eine an der Y-Luerkupplung 76 am proximalen Ende eingeleitete Steuerbewegung verlustarm bis zum distalen Ende auf einen über dem Ballon 74 mit dem Implantationswerkzeug 70 gekoppelten Elektrodenträgerkörper zu übertragen.

Um mit Hilfe des Implantationswerkzeuges 70 eine Stimulationselektrodenleitung 10 zu implantieren, erfolgt zunächst eine venöse Punktion an geeigneter Stelle. Anschließend wird ein Führungsdraht gelegt, das heißt, mit seinem distalen Ende so weit vorgeschoben, bis das distale Ende jenen Ort passiert hat, an dem die Elektrodenträgerkörper 16 und 20 der Stimulationselektrodenleitung 10 platziert werden sollen. Anschließend wird das Implantationswerkzeug 70 auf den Führungsdraht aufgeschoben. Dazu dient das Lumen im Inneren des inneren Schubschlauches 72a. Das Implantationswerkzeug 70 und die Stimulationselektrodenleitung 10 sind dabei vorzugsweise im Bereich des distalen Elektrodenträgerkörpers 20 über den aufgepumpten Ballon 74 miteinander gekoppelt. Anschließend kann das Implantationswerkzeug 70 samt gekoppelter Stimulationselektrodenleitung 10 entlang des Führungsdrahtes vorgeschoben werden.

Sollte die Stimulationselektrodenleitung 10 bereits in einem Blutgefäß positioniert sein und der Implantationsort der Elektrodenträgerkörper verändert werden, wird der Führungsdraht 32 zunächst durch die Längsöffnungen der beiden Elektrodenträgerkörper hindurchgeführt. Anschließend wird das Implantationswerkzeug mit deflatiertem Ballon so weit vorgeschoben, bis der deflatierte Ballon beispielsweise eine gewünschte Position innerhalb der Längsöffnung im distalen Elektrodenträgerkörper erreicht hat. Der Ballon 74 wird dann aufgepumpt, um den distalen Elektrodenträgerkörper mit dem Implantationswerkzeug zu kuppeln. Anschließend kann die Position des distalen Elektrodenträgerkörpers verändert werden.

Die Position des proximalen Elektrodenträgerkörpers relativ zur Position des distalen Elektrodenträgerkörpers und somit der Grad der Streckung der Leitungsbrücke können eingestellt werden, indem der Ballon im deflatierten Zustand in die Längsöffnung im proximalen Elektrodenträgerkörper eingeführt und dort expandiert wird. Anschließend kann der proximale Elektrodenträgerkörper entsprechend positioniert werden. Wenn beide Elektrodenträgerkörper den gewünschten Implantationsort eingenommen haben, kann das Implantationswerkzeug 70 nach Deflatieren des Ballons entfernt werden.

Die Oberflächen der Längsöffnungen in den Elektrodenträgerkörpern sowie die Oberfläche des Ballons sind dabei so gestaltet, dass zum einen ein sicheres Ankoppeln des Ballons an den jeweiligen Elektrodenträgerkörper möglich ist, wenn der Ballon expandiert ist. Auf der anderen Seite soll der Ballon im deflatierten Zustand möglichst reibungsarm durch die Längsöffnungen der Elektrodenträgerkörper und durch das entsprechende Lumen der Stimulationselektrodenleitung hindurchgeführt werden können. Gleichermaßen soll die Reibung zwischen dem vom inneren Schubschlauch 72a eingeschlossenen Lumen und dem Führungsdraht 32 möglichst gering sein.

## Patentansprüche

1. Stimulationselektrodenleitung (10) zum Anschluss an ein Elektrotherapiegerät wie einen Herzschrittmacher, einen Defibrillator oder dergleichen, wobei die Stimulationselektrodenleitung
ausgebildet ist, intraluminal in einem Blutgefäß oder im Herzen eines Patienten angeordnet zu werden,
und ein proximales und ein distales Ende aufweist,
sowie einen Elektrodenleitungsstecker (14) am proximalen Ende der Stimulationselektrodenleitung und Stimulationselektroden im Bereich des distalen Endes der Stimulationselektrodenleitung, welche eine elektrisch leitende Außenoberfläche aufweisen und elektrisch mit dem Elektrodenleitungsstecker verbunden sind, wobei
- die Stimulationselektrodenleitung im Bereich ihres distalen Endes wenigstens einen distalen Elektrodenträgerkörper (20) aufweist, der wenigstens eine Elektrode (24) trägt und über eine Leitungsbrücke (22) mit einem distalen Ende eines ersten, gestreckten Elektrodenleitungsabschnitts (12) verbunden ist, wobei die Leitungsbrücke (22) derart vorgeformt ist, dass sie im entspannten Zustand nicht gestreckt und elastisch streckbar ist,
- der erste, gestreckte Elektrodenleitungsabschnitt (12) ein Lumen aufweist, welches am distalen Ende des ersten, gestreckten Elektrodenleitungsabschnitts (12) in einer distalen Öffnung, durch die ein Führungsdraht aus der Stimulationselektrodenleitung (10) austreten kann, mündet, und
- der distale Elektrodenträgerkörper (20) eine Längsöffnung aufweist, durch die ein distales Ende eines Führungsdrahtes in den distalen Elektrodenträgerkörper (20) eintreten kann,
**dadurch gekennzeichnet, dass**
am distalen Ende des ersten, gestreckten Elektrodenleitungsabschnitts ein proximaler Elektrodenträgerkörper (16) angeordnet ist, der die distale Öffnung aufweist, durch die ein Führungsdraht austreten kann und die Längsöffnung im distalen Elektrodenträgerkörper (20) dergestalt ist, dass ein Führungsdraht durch sie hindurchtreten kann.

2. Stimulationselektrodenleitung nach Anspruch 1, **dadurch gekennzeichnet, dass** die Leitungsbrücke wenigstens einen elektrischen Leiter (44) umfasst, der von einer elastischen, vorgeformten Kunststoffhülle (48) umgeben ist und die Elektrode auf dem distalen Elektrodenkörper kontaktiert.

3. Stimulationselektrodenleitung nach Anspruch 1 oder 2, **dadurch gekennzeichnet, dass** die Leitungsbrücke (22) schlangenförmig vorgeformt ist.

4. Stimulationselektrodenleitung nach Anspruch 1 oder 2, **dadurch gekennzeichnet, dass** die Leitungsbrücke (22) helixförmig vorgeformt ist.

5. Stimulationselektrodenleitung nach einem der Ansprüche 1 bis 4, **dadurch gekennzeichnet, dass** wenigstens der proximale Elektrodenträgerkörper (16) oder vorzugsweise beide Elektrodenträgerkörper (16, 20) einen größeren Außendurchmesser aufweisen als wenigstens der unmittelbar an den proximalen Elektrodenträgerkörper anschließende Abschnitt des ersten, gestreckten Elektrodenleitungsabschnitts (12).

6. Stimulationselektrodenleitung nach einem der Ansprüche 1 bis 5, **dadurch gekennzeichnet, dass** die Leitungsbrücke (22) einen geringeren Außendurchmesser hat als wenigstens der unmittelbar an den proximalen Elektrodenträgerkörper (16) anschließende Abschnitt des ersten, gestreckten Elektrodenleitungsabschnitts (12).

7. Stimulationselektrodenleitung nach einem der Ansprüche 1 bis 6, **dadurch gekennzeichnet, dass** beide Elektrodenträgerkörper (16, 20) annähernd den gleichen Außendurchmesser haben.

8. Stimulationselektrodenleitung nach einem der Ansprüche 1 bis 7, **dadurch gekennzeichnet, dass** die distale Öffnung in dem proximalen Elektrodenträgerkörper (16) einen größeren Innendurchmesser aufweist, als die Längsöffnung in dem distalen Elektrodenleitungskörper (20).

9. Stimulationselektrodenleitung nach einem der Ansprüche 1 bis 8, **dadurch gekennzeichnet, dass** die Elektrodenträgerkörper (16, 20) als Kunststoff- oder Keramikkörper ausgebildet sind.

10. Stimulationselektrodenleitung nach einem der Ansprüche 1 bis 9, **dadurch gekennzeichnet, dass** der oder die Elektrodenträgerkörper (16, 20) eine Länge von 1 bis 3 mm aufweisen.

11. Stimulationselektrodenleitung nach einem der Ansprüche 1 bis 10, **dadurch gekennzeichnet, dass** ein distaler Endbereich des ersten, gestreckten Elektrodenleitungsabschnitts (12), der oder die Elektrodenträgerkörper (16, 20) und die Leitungsbrücke (22) zur Anordnung im Koronarsinus oder einer vom Koronarsinus abzweigenden Lateralvene geeignet ausgebildet sind.

12. Stimulationselektrodenleitung nach einem der Ansprüche 1 bis 11, **dadurch gekennzeichnet, dass** die Leitungsbrücke (22) einen deutlich geringeren Durchmesser als der distale Elektrodenträgerkörper (20) hat und in Bezug auf eine Mittellängsachse der Stimulationselektrodenleitung (10) seitlich neben der distalen Öffnung im proximalen Elektrodenleitungsabschnitt (12) an den proximalen Elektrodenleitungsabschnitt anschließt.

13. Stimulationselektrodenleitung nach einem der Ansprüche 1 bis 12, **dadurch gekennzeichnet, dass** die Leitungsbrücke (22) seitlich neben der Längsöffnung im distalen Elektrodenträgerkörper (20) an den distalen Elektrodenträgerkörper anschließt.

14. Implantationswerkzeug und Stimulationselektrodenleitung nach einem der Ansprüche 1 bis 13, **dadurch gekennzeichnet, dass** das Implantationswerkzeug (70) mindestens einen Schubschlauch mit einem Lumen für einen Führungsdraht umfasst, so dass der Schlauch über einen Führungsdraht zu führen ist, wobei der Schlauch derart gestaltet ist, dass er eine ausreichende Festigkeit besitzt und mit wenigstens einem Elektrodenträgerkörper (16, 20) zu koppeln ist, um den Elektrodenträgerkörper mittels des Schlauches in einem Blutgefäß vorschieben zu können.

## Claims

1. A stimulation electrode lead (10) for connecting to an electrotherapy device such as a cardiac pacemaker, a defibrillator or the like, wherein the stimulation electrode lead
is configured for intraluminal placement within a blood vessel or within the heart of a patient,
and comprises a proximal end and a distal end,
and also an electrode lead plug (14) at the proximal end of the stimulation electrode lead and stimulation electrodes in the region of the distal end of the stimulation electrode lead, which stimulation electrodes have an electrically conductive outer surface and are electrically connected to the electrode lead plug, wherein
- the stimulation electrode lead comprises, in the region of its distal end, at least one distal electrode support member (20), which supports at least one electrode (24) and is connected by a wire bridge (22) to a distal end of a first, extended electrode lead portion (12), wherein the wire bridge (22) is pre-formed such that, when in a relaxed state, the wire bridge is not extended and can be elastically extended,
- the first, extended electrode lead portion (12) comprises a lumen, which leads at the distal end of the first, extended electrode lead portion (12) into a distal opening through which a guide wire can exit the stimulation electrode lead (10), and
- the distal electrode support member (20) comprises a longitudinal opening through which a distal end of a guide wire can enter the distal electrode support member (20),
**characterised in that**
a proximal electrode support member (16) is arranged at the distal end of the first, extended electrode lead portion and comprises a distal opening through which a guide wire can exit, and the longitudinal opening in the distal electrode support member (20) is formed such that a guide wire can pass through it.

2. The stimulation electrode lead according to claim 1, **characterised in that** the wire bridge comprises at least one electrical conductor (44), which is surrounded by an elastic, pre-formed plastic sleeve (48) and which contacts the electrode on the distal electrode support member.

3. The stimulation electrode lead according to claim 1 or 2, **characterised in that** the wire bridge (22) is pre-formed in a snake-like shape.

4. The stimulation electrode lead according to claim 1 or 2, **characterised in that** the wire bridge (22) is pre-formed in a helical shape.

5. The stimulation electrode lead according to any one of claims 1 to 4, **characterised in that** at least the proximal electrode support member (16) or preferably both electrode support members (16, 20) has/have a larger outer diameter than at least the portion of the first, extended electrode lead portion (12) which directly adjoins the proximal electrode support member.

6. The stimulation electrode lead according to any one of claims 1 to 5, **characterised in that** the wire bridge (22) has a smaller outer diameter than at least the portion of the first, extended electrode lead portion (12) which directly adjoins the proximal electrode support member (16).

7. The stimulation electrode lead according to any one of claims 1 to 6, **characterised in that** both electrode support members (16, 20) have approximately the same outer diameter.

8. The stimulation electrode lead according to any one of claims 1 to 7, **characterised in that** the distal opening in the proximal electrode support member (16) has a larger inner diameter than the longitudinal opening in the distal electrode lead member (20).

9. The stimulation electrode lead according to any one of claims 1 to 8, **characterised in that** the electrode support members (16, 20) are formed as plastic or ceramic members.

10. The stimulation electrode lead according to any one of claims 1 to 9, **characterised in that** the electrode support member(s) (16, 20) has/have a length of from 1 to 3 mm.

11. The stimulation electrode lead according to any one of claims 1 to 10, **characterised in that** a distal end region of the first, extended electrode lead portion (12), the electrode support member(s) (16, 20) and the wire bridge (22) are suitably configured for arrangement in the coronary sinus or a lateral vein branching off from the coronary sinus.

12. The stimulation electrode lead according to any one of claims 1 to 11, **characterised in that** the wire bridge (22) has a much smaller diameter than the distal electrode support member (20) and adjoins the proximal electrode lead portion laterally with respect to a central longitudinal axis of the stimulation electrode lead (10), adjacently to the distal opening in the proximal electrode lead portion (12).

13. The stimulation electrode lead according to any one of claims 1 to 12, **characterised in that** the wire bridge (22) adjoins the distal electrode support member laterally, adjacently to the longitudinal opening in the distal electrode support member (20).

14. An implantation tool and stimulation electrode lead according to any one of claims 1 to 13, **characterised in that** the implantation tool (70) comprises at least one pushing tube with a lumen for a guide wire, such that the tube is guided by a guide wire, wherein the tube is formed such that it has sufficient strength and can be coupled to at least one electrode support member (16, 20) in order to be able to advance the electrode support member in a blood vessel by means of the tube.

## Revendications

1. Sonde de stimulation (10) pour le branchement sur un appareil d'électrothérapie comme un stimulateur cardiaque, un défibrillateur ou similaire, où la sonde de stimulation
est conçue pour être disposée de manière intraluminale dans un vaisseau sanguin ou dans le coeur d'un patient,
et présente une extrémité proximale et une extrémité distale,
ainsi qu'un connecteur de sonde (14) à l'extrémité proximale de la sonde de stimulation et des électrodes de stimulation dans la zone de l'extrémité distale de la sonde de stimulation, lesquelles présentent une surface extérieure électriquement conductrice et sont reliées électriquement avec le connecteur de la sonde, où
- la sonde de stimulation présente au moins un corps de support d'électrode (20) distal dans la zone de son extrémité distale, qui porte au moins une électrode (24) et est relié avec une extrémité distale d'une première section de sonde (12) allongée par l'intermédiaire d'un pontage de ligne (22), où le pontage de ligne (22) est préformé de telle manière qu'il n'est pas allongé à l'état détendu et peut être allongé de manière élastique,
- la première section de sonde (12) allongée présente une lumière, laquelle peut sortir à l'extrémité distale de la première section de sonde (12) allongée, débouche dans un orifice distal, par lequel un fil de guidage peut s'échapper de la sonde de stimulation (10), et
- le corps de support d'électrode (20) distal présente un orifice longitudinal par lequel une extrémité distale d'un fil de guidage peut pénétrer dans le corps de support d'électrode (20) distal,
**caractérisée en ce**
**qu'**à l'extrémité distale de la première section de sonde allongée est disposé un corps de support d'électrode (16) proximal, qui présente l'orifice distal, par lequel un fil de guidage peut sortir et l'ouverture longitudinale dans le corps de support d'électrode (20) distal est conçue de telle sorte qu'un fil de guidage puisse le traverser.

2. Sonde de stimulation selon la revendication 1, **caractérisée en ce que** le pontage de ligne comprend au moins un conducteur (44) électrique qui est entouré d'une gaine en matière plastique (48) préformée élastique et l'électrode est en contact sur le corps d'électrode distal.

3. Sonde de stimulation selon la revendication 1 ou 2, **caractérisée en ce que** le pontage de lignes (22) est préformé en forme de serpent.

4. Sonde de stimulation selon la revendication 1 ou 2, **caractérisée en ce que** le pontage de lignes (22) est préformé en forme d'hélice.

5. Sonde de stimulation selon l'une des revendications 1 à 4, **caractérisée en ce qu**'au moins le corps de support d'électrode (16) proximal ou de préférence, les deux corps de support d'électrode (16, 20), présentent un diamètre extérieur plus grand qu'au moins la partie immédiatement adjacente au corps de support d'électrode de la première section de sonde (12) allongée.

6. Sonde de stimulation selon l'une des revendications 1 à 5, **caractérisée en ce que** le pontage de lignes (22) a un diamètre extérieur plus faible qu'au moins la partie immédiatement adjacente au corps de support d'électrode (16) proximal de la première section de sonde (12) allongée.

7. Sonde de stimulation selon l'une des revendications 1 à 6, **caractérisée en ce que** les deux corps de support d'électrode (16, 20) ont un diamètre à peu près identique.

8. Sonde de stimulation selon l'une des revendications 1 à 7, **caractérisée en ce que** l'orifice distal dans le corps de support d'électrode (16) proximal présente un diamètre intérieur plus grand que l'orifice longitudinal dans le corps de sonde (20) distal.

9. Sonde de stimulation selon l'une des revendications 1 à 8, **caractérisée en ce que** les corps de support d'électrode (16, 20) sont conçus sous la forme de corps en matière plastique ou en céramique.

10. Sonde de stimulation selon l'une des revendications 1 à 9, **caractérisée en ce que** le ou les corps de support d'électrode (16, 20) présentent une longueur de 1 à 3 mm.

11. Sonde de stimulation selon l'une des revendications 1 à 10, **caractérisée en ce qu**'une zone terminale distale de la première section de sonde (12) allongée du ou des corps de support d'électrode (16, 20) et le pontage de lignes (22) sont conçus appropriés pour l'agencement dans le sinus coronaire ou dans une veine latérale partant du sinus coronaire.

12. Sonde de stimulation selon l'une des revendications 1 à 11, **caractérisée en ce que** le pontage de lignes (22) a un diamètre nettement inférieur au corps de support d'électrode (20) distal et se raccorde à la section de sonde proximale par rapport à un axe central de la sonde de stimulation (10) latéralement à côté de l'orifice distal dans la section de sonde (12) proximale.

13. Sonde de stimulation selon l'une des revendications 1 à 12, **caractérisée en ce que** le pontage de lignes (22) se raccorde au corps de support d'électrode distal latéralement à côté de l'orifice longitudinal dans le corps de support d'électrode (20) distal.

14. Outil d'implantation et sonde de stimulation selon l'une des revendications 1 à 13, **caractérisés en ce que** l'outil d'implantation (70) comprend au moins un tuyau coulissant avec une lumière pour un fil de guidage, de sorte que le tuyau est à guider par l'intermédiaire d'un fil de guidage, où le tuyau est conçu de telle manière qu'il possède une résistance suffisante et est à coupler avec au moins un corps de support d'électrode (16, 20), afin de pouvoir pousser vers l'avant le corps de support d'électrode au moyen du tuyau dans un vaisseau sanguin.
